# EUROPÄISCHE PATENTSCHRIFT

(11) **EP 3 930 595 B1**
(45) Veröffentlichungstag und Bekanntmachung des Hinweises auf die Patenterteilung: **25.01.2023**
(21) Anmeldenummer: 20703008.1
(22) Anmeldetag: 30.01.2020
(51) Int. Cl.: A61B 17/15

(54) **FIXIERUNGSVORRICHTUNG FÜR EIN TIBIA-AUSRICHTUNGSSYSTEM**
FIXING DEVICE FOR A TIBIA ALIGNMENT SYSTEM
DISPOSITIF DE FIXATION POUR UN SYSTÈME D'ALIGNEMENT DE TIBIA

(30) Priorität: 27.02.2019 DE 102019104965
(43) Veröffentlichungstag der Anmeldung: 05.01.2022
(73) Patentinhaber: Aesculap AG, 78532 Tuttlingen/Donau (DE)
(72) Erfinder: BÖTTIGER, Roland, 78604 Rietheim-Weilheim (DE)
(74) Vertreter: Winter, Brandl - Partnerschaft mbB
(86) Internationale Anmeldenummer: PCT/EP2020/052324
(87) Internationale Veröffentlichungsnummer: WO 2020/173657

(56) Entgegenhaltungen:
- EP-A1- 1 579 812
- US-A- 5 628 749
- US-A1- 2006 200 159

## Beschreibung

### Technischer Hintergrund

Die vorliegende Erfindung betrifft eine Fixierungsvorrichtung für ein Tibia-Ausrichtungssystem bei Knieoperationen mit einem Querbalken, welcher an seinem vorderen Ende mit einer zur Ausrichtung entlang einer Tibia vorgesehenen Hauptstange des Tibia-Ausrichtungssystems verbindbar ist, und an einem hinteren Ende ein Ankersystem mit zumindest einem Ankerstift aufweist, der schräg, insbesondere quer, zu dem Querbalken verläuft und in ein kniegelenkseitiges Ende der Tibia einschlagbar ist.

Bei Knieoperationen wird zunächst ein Tibia-Ausrichtungssystem, z.B. ein Extramedulläres Tibia-Ausrichtungsinstrument (ETA), über eine fußseitige Fixierung und eine knieseitige Fixierung befestigt, sodass eine Hauptstange (Einstellstange) entlang eines Schienbeins oder einer Tibia eines Patienten ausgerichtet ist. Diese Ausrichtung wird im Rahmen der Anmeldung als senkrecht oder vertikal bezeichnet, was lediglich die Beschreibung vereinfachen soll und sich nicht auf eine Ausrichtung im Raum bezieht. Gleichermaßen werden nachfolgend die Begriffe "vorne" als der Hauptstange des Tibia-Ausrichtungssystems zugewandt und "hinten" als dieser abgewandt verwendet. Anschließend kann eine an der Hauptstange angebundene bzw. gehaltene Sägeführung oder Sägelehre an dem Knie über das Tibia-Ausrichtungssystem positioniert und ausgerichtet werden. Das heißt, das Tibia-Ausrichtungssystem ist unten (d.h., entlang der Hauptstange an einem fußseitigen Ende derselben) mit beispielsweise einer Fußklammer (fußseitige Fixierung) und oben (d.h., entlang der Hauptstange an einem knieseitigen Ende derselben) über den Querbalken als C-Bogen mit der Tibia verbunden. Zur Einstellung der Schnitttiefe wird anschließend die Sägelehre / Sägeführung relativ zum gesamten Haltekonstrukt (C-Bogen) in vertikaler Richtung bzw. parallel zur Tibia oder entlang der Hauptstange verschoben bzw. eingestellt. Dies kann über eine Rasterung oder eine Gewindebestellung erfolgen. Zum Durchführen eines Schnitts mithilfe der Sägeführung oder Sägelehre muss diese an der Tibia befestigt werden und anschließend das Tibia-Ausrichtungssystem wieder entfernt werden.

### Stand der Technik

Die knieseitige Fixierung des Tibia-Ausrichtungssystems kann bekanntermaßen auf in Wesentlichen zwei Arten erfolgen. Zum einen kann ausgehend von vorne (d. h. von anterior oder ausgehend von einer einer Hauptstange des Tibia-Ausrichtungssystems zugewandten Seite) ein Pin durch ein Befestigungsloch in der angelegten Sägelehre oder Sägeführung geschoben werden. Um nachträglich eine Schnitttiefe einstellen zu können, muss das Befestigungsloch als Langloch ausgeführt sein. Innerhalb dieses Langlochs kann dann die Schnitttiefeneinstellung über Rastungen oder über eine Gewindemutter oder eine Klemmschraube in vertikaler/senkrechter Richtung oder entlang der Hauptstange oder in Richtung der Schnitttiefe vorgenommen werden. Zum anderen kann die knieseitige Fixierung mittels eines Querbalkens von oben (proximal), in einer Stirnseite bzw. in einer Emenentia der Tibia an dem Kniegelenk erfolgen. Dazu ragen Beispielsweise zwei Pins/Stifte aus dem Querbalken, welche mit der senkrechten Hauptstange des Tibia-Ausrichtungssystems verbunden ist. Der Querbalken bzw. die vorstehenden Pins am Querbalken werden in die Eminentia eingeschlagen. Alternativ können auch Pins/Stifte durch den Querbalken geschraubt oder geschlagen werden.

Ein Problem bei vorstehend beschriebenen Lösungen ist das Einbringen der Fixierpins/-stifte. Werden Schraubpins/-stifte verwendet, besteht die Gefahr, dass zu lange Pins verwendet werden, welche beim Eintreiben in die Tibia, insbesondere motorisch angetrieben, Blutgefäße verletzen können. Um das Risiko zu langer Pins zu vermeiden, wird üblicherweise der Querbalken mit den vorstehenden Pins mit vorgegebener Länge verwendet beziehungsweise eingeschlagen.

Nach der Fixierung des Tibia-Ausrichtungssystems erfolgen wie bereits beschrieben die Einstellung der Schnitttiefe und die Fixierung der Sägelehre an der Tibia. Zur Ausführung eines tibialen Schnitts müssen jedoch die oberen/stirnseitigen (proximalen) Pins aus der Eminentia wieder entfernt werden. Hier liegt ein weiteres
Problem: Da letztlich die Sägelehre/Sägeführung verstellbar (über eine Rasterung oder Gewindeverstellung) mit dem gesamten Haltekonstrukt verbunden ist, muss diese Verbindung gelöst werden, wenn man den Querbalken beziehungsweise dessen vorstehende Pins aus der Eminentia zieht. Ansonsten wird an der bereits fixierten Sägelehre/Sägeführung gezogen. Im ungünstigsten Fall erfolgt das Lösen der Verbindung gleichzeitig mit dem Entfernen des Querbalkens.

Beispielsweise ist aus US 7 344 542 B2 eine Tibia-Ausrichtungsführung bekannt, bei welcher der Querbalken starr mit der Hauptstange verbunden ist und einen Hebel aufweist, welche bei einer Betätigung gegen die Tibia drückt, um die an dem Querbalken angebrachten Stifte aus der Tibia zu ziehen. Das heißt, der gesamte Querbalken mit den integrierten Pins wird gegen den Knochen ausgehebelt, wobei die Verbindung der Sägelehre zur senkrechten Hauptstange in nachteiliger Weise gleichzeitig mit gelöst werden muss.

Ferner ist aus US 2010/0087831 A1 ein chirurgischer Nagelzieher bekannt, welcher unter einen Nagelkopf greifen und diesen mittels eines Hebels und einem Getriebe aus dem Knochen ziehen kann.

Ferner offenbaren die US 6 090 114 A bzw. die US 7 344 542 B2 eine Fixierungsvorrichtung für ein Tibia-Ausrichtungssystem bei Knieoperationen nach dem Oberbegriff des vorliegenden Anspruchs 1. Dabei weist die Fixierungsvorrichtung einen Querbalken auf, welcher an seinem vorderen Ende mit einer zur Ausrichtung entlang einer Tibia vorgesehenen Hauptstange des Tibia-Ausrichtungssystems verbindbar ist und an einem hinteren Ende ein Ankersystem mit zumindest einem Ankerstift aufweist, der schräg zu dem Querbalken verläuft und in ein kniegelenkseitiges Ende der Tibia einschlagbar ist.

EP 1 579 812 A1 offenbart ein Gerät zum Positionieren einer Schnittlehre für die Tibiaresektion. Dabei hat das Gerät einen transversal angeordneten Arm, der verankert ist an einer Tibiaplattform, die mit dem Gerät verbunden ist, so dass ein Transaktionsblock relativ zur Armhöhe auf einer Geraden eingestellt wird. Für die Führung ist eine Gelenkanordnung vorgesehen, um unterschiedliche Relativpositionen
zwischen Führung, Arm und Vorrichtung zu realisieren. Die Anordnung weist zwei Schwenkachsen auf, die in anteriorer bzw. mittiger lateraler Richtung orientiert sind.

US 5 628 749 A offenbart ein Verfahren zum Präparieren der proximalen Tibia eines Patienten mit einer chirurgischen Säge und einem Schneidinstrument. Das Verfahren bereitet das proximale Schienbein des Patienten vor, um ein Schienbeinimplantat aufzunehmen. Zunächst wird der Markraum des Patienten aufgebohrt und anschließend ein Markraumstab als Referenz in den Markkanal eingebracht. Oberhalb der proximalen Tibia des Patienten ist ein erstes Schneidinstrument an den Stab gebunden. Das erste Schneidinstrument umfasst einen Griffel, der verwendet wird, um die Schnitttiefe durch Referenzieren der Oberseite der proximalen Tibia einzustellen. Die proximale Tibia wird dann mit der Säge transversal geschnitten, indem die ersten Schnittführungsflächen mit der Säge verfolgt werden. Das erste Schneidinstrument wird zusammen mit seinem Griffel entfernt. Ein sekundäres Schneidinstrument wird dann auf dem Stab oberhalb der proximalen Tibia platziert. Das zweite Schneideinstrument weist in seiner Mitte eine Schneide zum Schneiden in Längsrichtung in die proximale Tibia auf. Ein zweiter Schnitt wird an der proximalen Tibia durchgeführt, indem das Sekundärschneidinstrument und eine Führungsfläche, die am oberen Ende der Schneider vorgesehen ist, die in die Mitte der proximalen Tibia eingreifen, verfolgt werden.

Die als nächstliegender Stand der Technik aufgefasste US 2006 / 0200159 A1 offenbart eine tibiale Ausrichtungsführungsanordnung zum Positionieren einer Resektionsführung, wobei die Anordnung einen Hauptschaft mit einem proximalen Ende und einem distalen Ende umfasst. Die Baugruppe umfasst auch einen Ankerarm mit einem Eingriffsende und einem Verbindungsende. Das Verbindungsende ist vorzugsweise am proximalen Ende des Hauptschafts befestigt und das Eingriffsende weist vorzugsweise mindestens einen sich davon nach außen erstreckenden Ankerstift auf. Der mindestens eine Verankerungsstift ist angepasst, um die Tibia-Ausrichtungsführungsanordnung an einem Tibiaplateau zu verankern, damit die Resektionsführung korrekt an der Tibia ausgerichtet werden kann. Die Baugruppe beinhaltet ferner einen Hebel, der ein Betätigungsende und einen Griff beinhaltet. Der Hebel ist schwenkbar mit dem Ankerarm verbunden, wodurch eine Bewegung des Hebels erfolgt, während das Betätigungsende an der Tibiaplateauoberfläche anliegt.

### Zusammenfassung der Erfindung

Es ist daher die Aufgabe der Erfindung, die Nachteile des Stands der Technik zu vermeiden oder zumindest zu reduzieren und insbesondere eine Fixierungsvorrichtung für ein Tibia-Ausrichtungssystem bereitzustellen, insbesondere eine obere/stirnseitige Fixierung, welche sowohl bei der Anbringung als auch der Entfernung einfach zu handhaben ist.

Die der Erfindung zugrunde liegende Aufgabe wird durch eine Fixierungsvorrichtung für ein Tibia-Ausrichtungssystem für Knieoperationen nach Anspruch 1 gelöst, mit einem Querbalken, welcher an seinem vorderen Ende mit einer zur Ausrichtung entlang einer Tibia vorgesehenen Hauptstange des Tibia-Ausrichtungssystems verbindbar ist, und an seinem hinteren Ende ein Ankersystem mit zumindest einem Ankerstift aufweist, der schräg zu dem Querbalken verläuft und in ein kniegelenkseitiges Ende der Tibia einschlagbar ist. Das Ankersystem weist ferner einen Einschlagamboss auf, in welchem der zumindest eine Ankerstift integriert ist und welcher dafür ausgelegt ist, von außen auf ihn einwirkende Schlagkräfte auf den zumindest einen Ankerstift für dessen Eintreiben in die Tibia zu übertragen, wofür der Ankerstift und der Einschlagamboss längsverschiebbar in dem Querbalken geführt/gehalten sind.

In anderen Worten wird eine Fixierungsvorrichtung mit einem Querbalken bereitgestellt, in welchem ein Einschlagamboss mit darin integrierten Stiften zum Einschlagen der Stifte in ein Schienbein/eine Tibia derart gelagert / gehalten ist, dass die Stifte durch eine externe, auf den Einschlagamboss wirkende Kraft in die Tibia getrieben werden können, ohne dabei den Querbalken zu bewegen oder zu verschieben. Entsprechend kann vermieden werden, Kräfte vom Ankersystem beim Lösen desselben über den Querbalken auf die Hauptstange zu übertragen und dabei gegebenenfalls die Sägelehre oder Sägeführung zu verschieben. Der Einschlagamboss kann unterschiedliche Formen aufweisen, wie z.B. eine Quaderform oder eine
Zylinderform. Vorzugsweise entfernt er sich in Stiftrichtung derart von dem Querbalken nach oben bzw. weg von dem zu operierenden Knie, dass er über die übrigen Bauteile der Fixierungsvorrichtung und gegebenenfalls in einer Operation über einen Oberschenkelknochen hinausragt, sodass ein oberes (d. h. ein der Tibia bei einer Operation abgewandtes) Ende des Einschlagambosses gut durch einen Hammer oder vergleichbaren Instrumenten erreichbar bzw. zugänglich ist.

Demgemäß kann vorteilhafter Weise die knieseitige Fixierung des Tibia-Ausrichtungssystems hergestellt und/oder gelöst werden, indem der Einschlagamboss und der zumindest eine Ankerstift nach oben aus der Tibia und zumindest teilweise dem Querbalken gezogen werden, ohne eine Hauptstange des Tibia-Ausrichtungssystems, an welcher eine Sägelehre/Sägeführung befestigt ist, zu bewegen. Entsprechend ist sowohl eine einfache, bequeme und sichere Handhabung der erfindungsgemäßen Fixierungsvorrichtung möglich. Insbesondere ist ferner die Halterung/Führung des zumindest einen Ankerstifts oder das Einschlagambosses derart gestaltet, dass diese unverlierbar an dem Querbalken gehalten bzw. beweglich angebunden sind.

Vorzugsweise ist durch einen Anschlag zwischen dem Einschlagamboss und dem Querbalken eine Einschlaglänge des zumindest einen Ankerstifts definiert. Als Anschlag kann beispielsweise eine Stirnseite oder eine Stufe des Einschlagambosses dienen. Dadurch kann vorteilhafter Weise gewährleistet werden, dass der Ankerstift nicht zu tief in die Tibia eingeschlagen werden kann, wodurch entsprechende Verletzungen von beispielsweise Blutgefäßen vermieden werden kann.

Vorzugsweise weist die Fixierungsvorrichtung ferner eine Hilfseinrichtung zum Lösen des Einschlagambosses und des zumindest einen Ankerstifts auf. Dabei ist als die Hilfseinrichtung bevorzugt ein Hebel bereitgestellt, welcher mittig an dem Querbalken gelagert oder abgestützt ist, und an einem Hubende mit dem zumindest einen Ankerstift oder dem Einschlagamboss gelenkig verbunden oder darin abgestützt ist, um diesen relativ zu dem Querbalken in Stiftlängsrichtung zu verschieben.

Anders ausgedrückt, ist der Hebel entweder an dem Einschlagamboss oder dem zumindest einen Ankerstift vorzugsweise unverlierbar angelenkt und stützt sich mittig an dem Querbalken ab; oder der Hebel ist mittig an dem Querbalken vorzugsweise unverlierbar angelenkt und stützt sich an dem Hubende an dem Einschlagamboss oder dem zumindest einen Ankerstift ab. Mit anderen Worten, kann entweder ein Anlenkpunkt des vorzugsweise unverlierbar angelenkten Hebels an dem Einschlagamboss oder dem zumindest einen Ankerstift angeordnet sein, wobei sich ein Abstützpunkt des Hebels, bei dessen Betätigung, mittig an dem Querbalken befindet. Oder es kann ein Anlenkpunkt des vorzugsweise unverlierbar angelenkten Hebels mittig an dem Querbalken angeordnet sein, wobei sich ein an dem Hubende des Hebels angeordneter Abstützpunkt des Hebels, bei dessen Betätigung, an dem Einschlagamboss oder dem zumindest einen Ankerstift befindet.

Grundsätzlich ist es auch möglich, einen gesondert vorgesehenen Hebel bereitzustellen, welcher als Einzelteil zwischen den Querbalken und einen Ansatz des Ankersystems klemmbar ist, um den zumindest einen Ankerstift aus der Tibia zu hebeln.

Auf diese Weise können durch den Hebel das Ankersystem mit dem zumindest einen Ankerstift und dem Einschlagamboss und der Querbalken voneinander weg gedrückt werden, wodurch der zumindest eine Ankerstift während einer Operation aus der Tibia gezogen wird. Dadurch wird vermieden, dass der Hebel sich an dem Knochen abstützt, weshalb kaum Kräfte auf den Patienten übertragen werden und diese Ausgestaltung gewebeschonend ist. Ferner sind aufgrund der Hebelwirkung des Hebels durch den Operateur nur geringe Kräfte aufzubringen, weshalb dieser weniger schnell ermüdet und ferner eine ruhige, gleichmäßige Entfernung der Ankerstifte möglich ist.

Vorzugsweise sind der Hebel und der Einschlagamboss an einer Oberseite des Querbalkens als einer zu dem zumindest einen Ankerstift anderen Seite angeordnet. Mit anderen Worten, kommen vorzugsweise sowohl der Anlenkpunkt als auch der Abstützpunkt des Hebels an der Oberseite bzw. in einem Bereich oberhalb der Oberseite des Querbalkens zu liegen. Dabei kann insbesondere der Hebel eingerichtet bzw. einrichtbar sein, dass bei dessen Betätigung zum Lösen des Einschlagambosses und des zumindest einen Ankerstifts ein Bewegungsbereich und/oder kinematische Freiheitsgrade des Hebels ausschließlich an einer Oberseite des Querbalkens und damit nicht an der dem zumindest einen Ankerstift zugewandten Seite des Querbalkens liegen. Unter den Begriff des Bewegungsbereiches fällt insbesondere eine sich entwickelnde (bzw. evolvierende) Bewegungslinie zumindest eines Kontaktpunktes zwischen dem Hebel und dem zu lösenden Einschlagamboss mit dem zumindest einen Ankerstift. Sprich, bei Betätigung des Hebels wird eine Überkreuzung des Hebels als Hilfseinrichtung zum Lösen des Einschlagambosses und des zumindest einen Ankerstifts bzw. eines Bereichs und/oder Teilabschnitts des Hebels mit dem Querbalken vermieden. Somit wird effektiv verhindert, dass sich der Hebel bzw. der Teilabschnitt des Hebels bei dessen Betätigung überhaupt in die Nähe des Patienten bewegen kann. Dadurch weist diese bevorzugte Ausführungsform den besonderen Vorteil auf, dass die Gefahr einer, insbesondere schmerzhaften und/oder traumatisierenden, Berührung und/oder mechanischen Krafteinwirkung des Patienten, insb. der Tibia, durch die Hilfseinrichtung bzw. den Hebel im Vorfeld bzw. konstruktionsbedingt vollständig vermieden wird.

Nach einem weiteren Aspekt der Erfindung weist der Querbalken an seinem vorderen Ende ein Führungselement auf, über welches der Querbalken balkenaxial frei verschiebbar, insbesondere nach vorne und hinten, anschlagfrei an/in der Hauptstange des Tibia-Ausrichtungssystems gelagert/gehalten ist. Insbesondere ist der Querbalken an der Hauptstange frei herausziehbar gelagert/gehalten oder lagerbar/haltbar. Beispielsweise ist hierfür an der Hauptstange ein Lagerauge bereitgestellt, in welchem der Querbalken über eine zylindrische Lagerfläche gehalten ist. Dies ermöglicht es, dass der Operateur während einer Operation den Querbalken der Fixierungsvorrichtung einfach in die Hauptstange des Tibia-Ausrichtungssystems einschieben und herausziehen kann, ohne an der Sägelehre/Sägehalterung zu ziehen. Entsprechend kann das Risiko, diese beim Entfernen des Tibia-Ausrichtungssystems zu verschieben, minimiert werden. Zudem wird dabei die Handhabung des Tibia-Ausrichtungssystems beim Entfernen und beim Anbringen der Fixierungsvorrichtung deutlich vereinfacht und beschleunigt.

Vorzugsweise ist durch die balkenaxial verschiebbare Lagerung des Querbalkens an/in der Hauptstange ein Abstand des zumindest einen Ankerstifts zu der Hauptstange einstellbar. Auf diese Weise kann nach dem Einschlagen der Pins die Hauptstange knieseitig durch den Querbalken geführt nach vorne verschoben werden, wodurch der für die Schnitthöhe verantwortliche Schnittblock (Sägelehre/Sägehalterung) von der Tibia beabstandet wird und anschließend kollisionsfrei mit der Tibia nach oben oder unten / entlang der Hauptstange bewegbar ist. Bei einem festen Abstand zwischen dem zumindest einen Ankerstift und der Hauptstange kann u. U. der Schnittblock auf der Tuberositas der Tibia auflaufen und diese ggf. verletzen.

Nach einer bevorzugten Ausführungsform sind zwei parallel zueinander verlaufende, insbesondere entlang des Querbalkens versetzte, Ankerstifte vorgesehen, welche mit dem Einschlagamboss verbunden sind. Dadurch wird eine Fixierung des Ankersystems an der Tibia verbessert.

In anderen Worten ausgedrückt, wird die der Erfindung zu Grunde liegende Aufgabe gelöst durch eine proximale (obere/stirnseitige) Fixierung (Extramedulläres Tibia Ausrichtungsinstrument - ETA) das (als Fixierungsvorrichtung) einen mehrteiligen Querbalken (mit zueinander beweglichen, separaten Bauteilen) aufweist, welche nach dem Anlegen des ETA von der Seite des Operateurs aus eingeschoben werden kann. Der Querbalken bleibt in anterior-posteriorer Richtung in der ETA beweglich (nach vorne und hinten verschiebbar), sodass er sich auf die jeweilige Größe bzw. die Tiefe des Tibia-Plateaus einstellen kann. Die Fixierung an der Tibia selbst erfolgt mittels zumindest einem Pin, vorzugsweise zwei Pins, welche in einem Amboss / Einschlagamboss integriert sind. Der Amboss ist so ausgelegt, dass er über den gebeugten Femur hinausragt und mit einem Hammer in die Eminentia getrieben werden kann. Zum Entfernen der Pins wird ein Hebel nach unten in Richtung des Querbalkens bewegt, welcher den Amboss mit den Pins ausgehebelt. Anschließend kann der mehrteilige Querbalken zum Operateur hinaus aus der ETA herausgezogen werden. Es erfolgt keinerlei Beeinflussung der Sägelehrenposition. Alle Einzelteile des Querbalkens sind vorzugsweise unverlierbar miteinander verbunden. Insbesondere ist der integrierte Einschlagamboss mit den Pins relevant. Der Querbalken ist nicht fix mit der ETA verbunden, sondern kann nach vorne oder hinten aus einer Führung geschoben werden sowie sich auf die Tiefe des Tibia-Plateaus einstellen. Der Querbalken beinhaltet auch einen Hebel zum Herausziehen der Pins, welche im Einschlagamboss integriert sind.

### Kurzbeschreibung der Figuren

Nachfolgend wird die vorliegende Erfindung anhand einer beispielhaften Ausführungsform mithilfe der beigefügten Figuren beschrieben. Diese sollen den Schutzumfang der vorliegenden Erfindung nicht begrenzen sondern lediglich der Anschauung dienen.
Fig. 1 zeigt eine Seitenansicht einer erfindungsgemäßen Fixierungsvorrichtung, und
Fig. 2 zeigt eine Rückansicht der Fixierungsvorrichtung aus Fig. 1.
Fig. 3 zeigt eine Seitenansicht der Fixierungsvorrichtung während einer Operation.

### Detaillierte Beschreibung der bevorzugten Ausführungsform

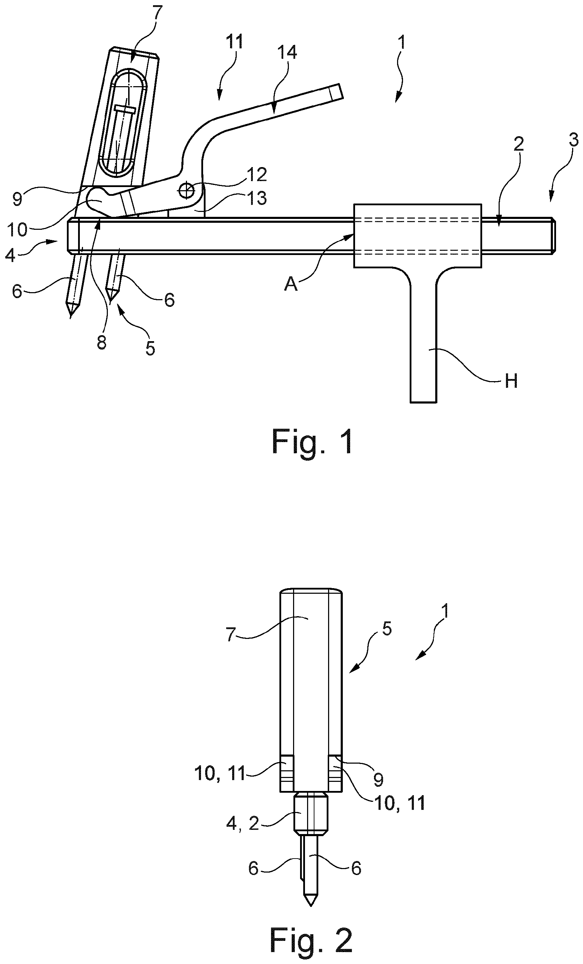

Die in Fig. 1 dargestellte Fixierungsvorrichtung 1 weist einen Querbalken 2 auf, an dessen vorderen Ende 3 (rechts in Fig. 1) eine vorzugsweise zylindrische Führung vorgesehen ist, über welche der Querbalken 2 in eine entsprechende Aufnahme Ader Hauptstange H eines Tibia-Ausrichtungssystems (ETA) eingeschoben werden kann. An einem hinteren Ende 4 (links in Fig. 1) des Querbalkens 2 ist ein Ankersystem 5 vorgesehen, welches zwei in Balkenlängsrichtung axial beabstandet angeordnete Ankerstifte 6 aufweist, die an einer Unterseite (in Fig.1 gesehen) des Querbalkens 2 schräg, vorzuweise in einem Winkel von 80° bis 130°, aus diesem herausragen.

Die Ankerstifte 6 sind oben / an einer der Tibia T abgewandten Seite (oben in Fig. 1) mit einem Einschlagamboss 7 verbunden / daran gehalten / damit integriert / befestigt, welcher an einer Oberseite des Querbalkens 2 angeordnet ist und sich in Stiftlängsrichtung nach oben erstreckt. Ferner sind die Ankerstifte 6 unterschiedlich lang ausgebildet, insbesondere ist der weiter hinten angeordnete Ankerstift 6 kürzer als der vorne angeordnete Ankerstift 6.

Eine untere / der Tibia T und dem Querbalken zugewandte Stirnseite des Einschlagambosses 7 ist derart angepasst, insbesondere angeschrägt, um in einer hier dargestellten eingeschlagenen Position des Ankersystems 4 an der Oberseite des Querbalkens 2 anzuliegen und somit einen Anschlag 8 zur Begrenzung einer Einschlagslänge der Ankerstifte 6 bereit zu stellen. Ferner bildet der Einschlagamboss 7 an seinem unteren Ende (unten in Fig. 1) eine Stufe 9 aus, an welcher ein Hubende 10 eines Hebels 11 angreift, um das Ankersystem 4 von dem Querbalken 2 wegzudrücken. Dazu drückt das abgerundete Hubende 10 gegen die Stufe 9. Dadurch fungiert der Hebel 11 als Hilfseinrichtung zum Lösen des Einschlagambosses 7 mit den zwei Ankerstiften 6, 6.

Der Hebel 11 ist S-förmig ausgebildet und erstreckt sich im Wesentlichen nach vorne. An einem mittigen Bereich der S-Form des Hebels 11 ist ein Scharnierstift 12 vorgesehen, welcher in korrespondierenden Scharnieraugen zur Ausbildung eines Anlenkpunktes steckt / einsteht, der in einem Fortsatz / Vorsprung 13 an der Oberseite des Querbalkens 2 ausgebildet ist. Der Scharnierstift 12 steht dabei senkrecht aber versetzt zu der Längsachse des Querbalkens 2.

Wie Fig. 1 zu entnehmen, sind der Hebel 11 und der Einschlagamboss 7 an der Oberseite des Querbalkens 2 angeordnet, wodurch sie auf einer zu den zwei Ankerstiften 6, 6 anderen (d.h. Tibia-abgewandten) Seite zu liegen kommen. Somit befinden sich sowohl der durch den Scharnierstift 12 in den korrespondierenden Scharnieraugen ausgebildete Anlenkpunkt des Hebels 11 als auch ein zwischen dem Hubende 10 und der Stufe 9 ausgebildeter Abstützpunkt des Hebels 11 an der Oberseite des Querbalkens 2.

Ein dem Hubende 10 gegenüberliegendes Griffende 14 des Hebels 11 dient als Griff, um den Hebel zu betätigen. Die S-Form des Hebels 11 gewährleistet, dass zwischen dem Griffende 14 und der Oberseite des Querbalkens 2 ausreichend Raum ist, um das Griffende 14 greifen und bewegen zu können. Bei Betätigung des Hebels 11 durch Herunterdrücken des Griffendes 14 in Richtung hin zu der Oberseite des Querbalkens 2 hebt sich wippenartig der Abstützpunkt in Richtung weg von der Oberseite des Querbalkens 2. Dabei kommt es zu keiner Überkreuzung der Bewegungslinie des Abstützpunktes mit der Längsachse des Querbalkens 2. Somit findet die Betätigung bzw. der kinematische Ablauf der Bewegung des Hebels 11 auf der zu den zwei Ankerstiften 6, 6 abgewandten, also der Tibia-abgewandten, Seite des Querbalkens 2 bzw. in einem Raum oberhalb der Oberseite des Querbalkens 2 statt.

Fig. 2 zeigt eine Rückansicht der vorstehend beschriebenen Fixierungsvorrichtung 1. In der Fig. 2 ist erkennbar, dass der Einschlagamboss 7, der Hebel 11, der Querbalken 2 und die Ankerstifte 6 symmetrisch zueinander bzw. symmetrisch zu einer Symmetrieebene, welche sich in Fig. 2 gesehen in vertikaler Richtung erstreckt, in einer Linie angeordnet sind. Insbesondere ist das gabelförmige Hubende 10 dargestellt, welches im Bereich der Stufe 9 um den Einschlagamboss 7 greift, um nach oben gegen die Stufe 9 zu drücken. An seiner Stirnseite 8 liegt der Einschlagamboss 7 an dem hinteren Ende 4 des Querbalkens 2 an, um die Einschlaglänge der Ankerstifte 6 zu begrenzen. Ferner ist der Querbalken 2 an einem hinteren Ende ausreichend schmal ausgebildet, sodass er zwischen den Zinken / Backen des gabelförmigen Hubendes 10 liegen kann. Dies gewährleistet eine einfache Handhabung und eine Montage des Hebels 11.

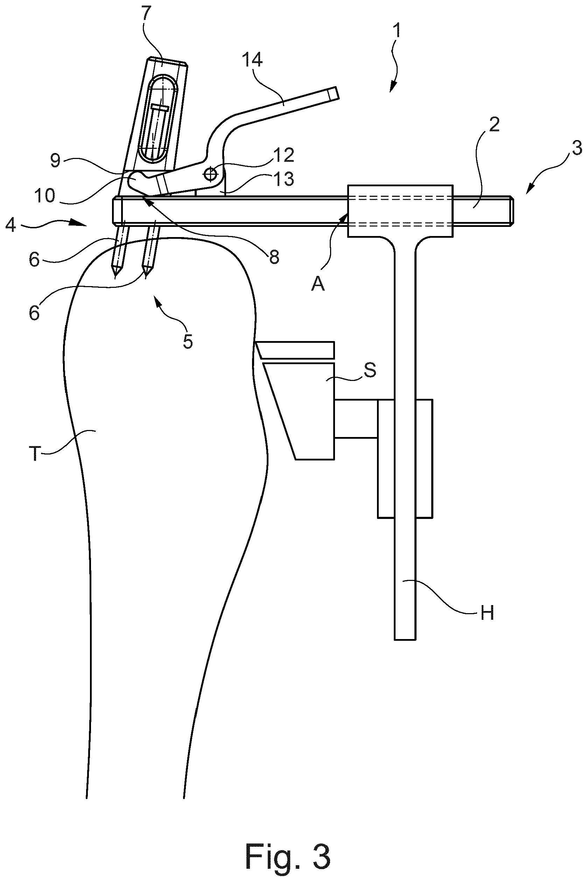

Die in Fig. 3 dargestellte Ansicht entspricht im Wesentlichen einer vereinfachten Darstellung der Ausführungsform der erfindungsgemäßen Fixierungsvorrichtung 1 aus Fig. 1, stellt die Fixierungsvorrichtung 1 jedoch während ihrer Verwendung bei einer Knieoperation dar. Genauer ist zu sehen, dass von oben (wie in Fig. 3 gesehen) bzw. stirnseitig die Ankerstifte 6, welche an dem hinteren Ende 4 des Querbalkens 2 gehalten sind, in eine obere Stirnseite der Tibia T eingeschlagen sind. Dadurch ist der Querbalken 2 an der Tibia T fixiert. Der Querbalken erstreckt sich nach vorne in Richtung der Hauptstange H und ist daran balkenaxial verschiebbar geführt.

An der sich im Wesentlichen parallel zu der Tibia T erstreckenden Hauptstange H ist ein Schnittblock S bzw. eine Sägelehre oder Sägehalterung entlang der Hauptstange H verschiebbar gehalten. Wenn nun der Schnittblock S nach unten verschoben werden soll, würde, wie in dieser schematischen Ansicht gut erkennbar, bei einer starren Verbindung der Hauptstange H und des Querbalkens 2 der Schnittblock an einem Teil der Tibia T auflaufen oder daran entlang schaben und diese beschädigen. Dadurch, dass jedoch die Hauptstange H oder zumindest deren oberes Ende entlang des fixierten Querbalkens 2 nach vorne verschiebbar ist, kann auf diese gemeinsam mit dem Schnittblock S von der Tibia T beabstandet werden. Anschließend ist der Schnittblock S entlang der Hauptstange H verschiebbar, ohne dass eine Gefahr besteht, die Tibia T zu verletzen.

Um nach Fixierung des Schnittblocks S die Pins aus der Stirnseite der Tibia T zu ziehen, kann der Hebel 14 nach unten gedrückt werden, um den Einschlagamboss 7 und die daran angebrachten/gehaltenen Ankerstifte 6 relativ zu der Tibia T und dem Querbalken 2 nach oben zu ziehen. Aufgrund der stiftaxialen Verschiebbarkeit der Ankerstifte 6 in dem Querbalken 2 können der Querbalken 2 und die Hauptstange H, wie vorstehend genauer beschrieben, unbeweglich bleiben und muss nicht gleichzeitig die die Verbindung des Schnittblocks S / der Sägelehre zu der Hauptstange H gelöst werden.

### Bezugszeichenliste

- 1: Fixierungsvorrichtung
- 2: Querbalken
- 3: Vorderes Ende des Querbalkens
- 4: Hinteres Ende des Querbalkens
- 5: Ankersystem
- 6: Ankerstift
- 7: Einschlagamboss
- 8: Anschlag
- 9: Stufe
- 10: Hubende
- 11: Hebel
- 12: Scharnierstift
- 13: Fortsatz
- 14: Griffende
- H: Hauptstange
- A: Aufnahme
- S: Schnittblock/Sägehalterung/Sägelehre
- T: Tibia

## Patentansprüche

1. Fixierungsvorrichtung (1) für ein Tibia-Ausrichtungssystem bei Knieoperationen mit
einem Querbalken (2), welcher an seinem vorderen Ende (3) mit einer zur Ausrichtung entlang einer Tibia vorgesehenen Hauptstange (H) des Tibia-Ausrichtungssystems verbindbar ist, und an einem hinteren Ende ein Ankersystem (5) mit zumindest einem Ankerstift (6) aufweist, der schräg zu dem Querbalken (2) verläuft und in ein kniegelenkseitiges Ende der Tibia einschlagbar ist,
wobei das Ankersystem (5) ferner einen Einschlagamboss (7) zur Verbesserung einer Fixierung des Ankersystems (5) an der Tibia aufweist, mit welchem der zumindest eine Ankerstift (6) verbunden und in welchem der zumindest eine Ankerstift (6) integriert ist und welcher dafür ausgelegt ist, von außen auf ihn einwirkende Schlagkräfte auf den zumindest einen Ankerstift (6) für dessen Eintreiben in die Tibia zu übertragen,
**dadurch gekennzeichnet, dass** dafür der Ankerstift (6) und der Einschlagamboss (7) längsverschiebbar in dem Querbalken (2) geführt sind.

2. Fixierungsvorrichtung (1) nach Anspruch 1, **wobei** durch einen Anschlag zwischen dem Einschlagamboss (7) und dem Querbalken (2) eine Einschlaglänge des zumindest einen Ankerstifts (6) definiert ist.

3. Fixierungsvorrichtung (1) nach einem der vorstehenden Ansprüche, **wobei** diese ferner eine Hilfseinrichtung (11) zum Lösen des Einschlagambosses (7) und des zumindest einen Ankerstifts (6) aufweist.

4. Fixierungsvorrichtung (1) nach Anspruch 3, **wobei** die Hilfseinrichtung ausgebildet ist als ein Hebel (11), welcher mittig an dem Querbalken (2) gelagert oder abgestützt ist, an einem Hubende (10) mit dem zumindest einen Ankerstift (6) oder dem Einschlagamboss (7) gelenkig verbunden oder darin abgestützt ist, um diesen relativ zu dem Querbalken (2) in Stiftlängsrichtung zu verschieben.

5. Fixierungsvorrichtung (1) nach einem der Ansprüche 3 oder 4, **wobei** die Hilfseinrichtung (11) als ein gesondert vorgesehener Hebel bereitgestellt ist, der als Einzelteil zwischen den Querbalken (2) und einen Ansatz des Ankersystems (5) klemmbar ist, um den zumindest einen Ankerstift (6) aus der Tibia zu hebeln.

6. Fixierungsvorrichtung (1) nach Anspruch 4, **wobei** der Hebel (11) an dem Einschlagamboss (7) oder dem zumindest einen Ankerstift (6), vorzugsweise unverlierbar, angelenkt ist und sich mittig an dem Querbalken (2) abstützt.

7. Fixierungsvorrichtung (1) nach Anspruch 4, **wobei** der Hebel (11) mittig an dem Querbalken (2), vorzugsweise unverlierbar, angelenkt ist und sich an dem Hubende (10) an dem Einschlagamboss (7) oder dem zumindest einen Ankerstift (6) abstützt.

8. Fixierungsvorrichtung (1) nach Anspruch 7, **wobei** der Hebel (11) und der Einschlagamboss (7) an einer Oberseite des Querbalkens (2) als einer zu dem zumindest einen Ankerstift (6) anderen Seite angeordnet sind.

9. Fixierungsvorrichtung (1) nach einem der Ansprüche 7 oder 8, **wobei:**
- eine untere, der Tibia und/oder dem Querbalken (2) zugewandte, Stirnseite des Einschlagambosses (7) derart angepasst, insbesondere angeschrägt, ist, um in einer eingeschlagenen Position des Ankersystems (4, 5) an der Oberseite des Querbalkens (2) zur Bereitstellung eines Anschlags (8) zur Begrenzung einer Einschlagslänge des zumindest einen Ankerstifts (6) anzuliegen;
- der Einschlagamboss (7) an seinem unteren Ende eine Stufe (9) zum Angreifen des, vorzugsweise abgerundeten, Hubendes (10) des Hebels (11) ausbildet, um das Ankersystem (4, 5) von dem Querbalken (2) wegzudrücken;
- der Hebel (11) S-förmig und sich im Wesentlichen nach vorne erstreckend ausgebildet und mittels eines Scharnierstiftes (12) in korrespondierenden, in einem Fortsatz und/oder Vorsprung (13) an der Oberseite des Querbalkens (2) ausgebildeten Scharnieraugen angelenkt ist; und
- ein dem Hubende (10) gegenüberliegendes Griffende (14) des Hebels (11) zu dessen Betätigung vorgesehen ist.

10. Fixierungsvorrichtung (1) nach einem der vorstehenden Ansprüche, **wobei** der Querbalken (2) an seinem vorderen Ende (3) ein Führungselement aufweist, über welches der Querbalken (2) balkenaxial frei verschiebbar an der Hauptstange (H) des Tibia-Ausrichtungssystems lagerbar ist.

11. Fixierungsvorrichtung (1) nach Anspruch 10, **wobei** der Querbalken (2) frei herausziehbar an der Hauptstange (H) lagerbar ist.

12. Fixierungsvorrichtung (1) nach einem der Ansprüche 10 oder 11, **wobei** durch die balkenaxial verschiebbare Lagerung des Querbalkens (2) ein Abstand des zumindest einen Ankerstifts (6) zu der Hauptstange (H) einstellbar ist, um eine Schnitttiefe einzustellen.

13. Fixierungsvorrichtung (1) nach einem der vorstehenden Ansprüche, **wobei** zwei parallel zueinander verlaufende Ankerstifte (6) vorgesehen sind, welche mit dem Einschlagamboss (7) verbunden sind.

## Claims

1. A fixing device (1) for a tibia alignment system for knee operations, comprising
a transverse bar (2) the front end (3) of which can be connected to a main rod (H) of the tibia alignment system, said main rod (H) being provided for alignment along a tibia, and a rear end of which has an anchor system (5) with at least one anchor pin (6) that extends obliquely to the transverse bar (2) and can be driven into a knee joint-side end of the tibia,
wherein, for improving fixation of the anchor system (5) to the tibia, the anchor system (5) further has a drive-in anvil (7) to which the at least one anchor pin (6) is connected and into which the at least one anchor pin (6) is integrated and which is designed to transmit impact forces acting on the drive-in anvil (7) from outside to the at least one anchor pin (6) in order to drive the same into the tibia,
**characterized in that** said anchor pin (6) and said drive-in anvil (7) are guided in the transverse bar (2) for this purpose in a longitudinally movable manner.

2. The fixing device (1) according to claim 1, **wherein** a drive-in length of the at least one anchor pin (6) is defined by a stop between the drive-in anvil (7) and the transverse bar (2).

3. The fixing device (1) according to any of the preceding claims, **wherein** said fixing device (1) further comprises an auxiliary device (11) for releasing the drive-in anvil (7) and the at least one anchor pin (6).

4. The fixing device (1) according to claim 3, **wherein** the auxiliary device is in the form of a lever (11) which is mounted or supported centrally on the transverse bar (2), is hinged, at one lifting end (10), to the at least one anchor pin (6) or the drive-in anvil (7) or supported therein so as to displace the latter relative to the transverse bar (2) in the longitudinal direction of the pin.

5. The fixing device (1) according to any one of claims 3 or 4, **wherein** the auxiliary device (11) is provided as a separately provided lever that can be clamped as single component part between the transverse bar (2) and a base of the anchor system (5) to lever the at least one anchor pin (6) out of the tibia.

6. The fixing device (1) according to claim 4, **wherein** the lever (11) is hinged, preferably captively, to the drive-in anvil (7) or the at least one anchor pin (6) and is supported centrally on the transverse bar (2).

7. The fixing device (1) according to claim 4, **wherein** the lever (11) is hinged, preferably captively, centrally to the transverse bar (2) and is supported at the lifting end (10) on the drive-in anvil (7) or the at least anchor pin (6).

8. The fixing device (1) according to claim 7, **wherein** the lever (11) and the drive-in anvil (7) are arranged on an upper side of the transverse bar (2) as a different side relative to the at least one anchor pin (6).

9. The fixing device (1) according to any one of the claims 7 or 8, **wherein:**
- a lower front face of the drive-in anvil (7) facing the tibia and/or the transverse bar (2) is adapted, particularly beveled, so as to abut, in a driven-in position of the anchor system (4, 5), on the upper side of the transverse bar (2) to provide a stop (8) for restricting a drive-in length of the at least one anchor pin (6);
- the drive-in anvil (7) forms a step (9) at its lower end for engaging the, preferably rounded, lifting end (10) of the lever (11) to force the anchor system (4, 5) away from the transverse bar (2);
- the lever (11) is designed to be S-shaped and to extend substantially forward and is hinged, by means of a hinge pin (12), in corresponding hinge eyes formed in an extension and/or a projection (13) on the upper side of the transverse bar (2); and
- a handle end (14) of the lever (11) opposed to the lifting end (10) is provided for actuation of the latter.

10. The fixing device (1) according to any one of the preceding claims, **wherein** the transverse bar (2) at its front end (3) includes a guide member via which the transverse bar (2) can be supported on the main rod (H) of the tibia alignment system to be freely movable along the bar axis.

11. The fixing device (1) according to claim 10, **wherein** the transverse bar (2) can be supported on the main rod (H) to be freely extractable.

12. The fixing device (1) according to any one of the claims 10 or 11, **wherein** a distance of the at least one anchor pin (6) from the main rod (H) can be adjusted by the support of the transverse bar (2) movable along the bar axis so as to adjust a cutting depth.

13. The fixing device (1) according to any one of the preceding claims, **wherein** two anchor pins (6) extending in parallel to each other are provided which are connected to the drive-in anvil (7).

## Revendications

1. Dispositif de fixation (1) pour un système d'alignement de tibia lors d'opérations du genou avec
une barre transversale (2), laquelle peut être reliée à son extrémité avant (3) à une tige principale (H) du système d'alignement de tibia prévue pour l'alignement le long d'un tibia, et présente en une extrémité arrière un système d'ancrage (5) avec au moins une cheville d'ancrage (6) qui se déroule en biais par rapport à la barre transversale (2) et peut être introduite dans une extrémité du tibia côté articulation du genou,
dans lequel le système d'ancrage (5) présente en outre une enclume d'impact (7) pour l'amélioration d'une fixation du système d'ancrage (5) contre le tibia, à laquelle enclume d'impact la au moins une cheville d'ancrage (6) est reliée et dans laquelle la au moins une cheville d'ancrage (6) est intégrée et laquelle enclume d'impact est destinée à transférer des forces de frappe qui agissent sur elle depuis l'extérieur à la au moins une cheville d'ancrage (6) pour son enfoncement dans le tibia,
**caractérisé en ce qu'**à cette fin, la cheville d'ancrage (6) et l'enclume d'impact (7) sont guidées dans la barre transversale (2) de manière longitudinalement coulissante.

2. Dispositif de fixation (1) selon la revendication 1, dans lequel une longueur d'impact de la au moins une cheville d'ancrage (6) est définie par un impact entre l'enclume d'impact (7) et la barre transversale (2).

3. Dispositif de fixation (1) selon l'une des revendications précédentes, dans lequel celui-ci présente en outre un équipement auxiliaire (11) pour le dégagement de l'enclume d'impact (7) et de la au moins une cheville d'ancrage (6).

4. Dispositif de fixation (1) selon la revendication 3, dans lequel l'équipement auxiliaire est conçu en tant que levier (11), lequel est monté ou s'appuie au centre contre la barre transversale (2), à une extrémité de course (10) est relié de manière articulée à la au moins une cheville d'ancrage (6) ou à l'enclume d'impact (7) ou s'appuie à l'intérieur de celles-ci, pour déplacer celle-ci relativement à la barre transversale (2) dans la direction longitudinale de la tige.

5. Dispositif de fixation (1) selon l'une des revendications 3 ou 4, dans lequel l'équipement auxiliaire (11) est fourni en tant que levier prévu séparément qui peut être coincé en tant que pièce détachée entre la barre transversale (2) et un épaulement du système d'ancrage (5) pour soulever la au moins une cheville d'ancrage (6) hors du tibia.

6. Dispositif de fixation (1) selon la revendication 4, dans lequel le levier (11) est articulé contre l'enclume d'impact (7) ou la au moins une cheville d'ancrage (6), de préférence de manière imperdable, et s'appuie au centre contre la barre transversale (2).

7. Dispositif de fixation (1) selon la revendication 4, dans lequel le levier (11) est articulé au centre contre la barre transversale (2), de préférence de manière imperdable, et s'appuie contre l'extrémité de course (10) contre l'enclume d'impact (7) ou la au moins une cheville d'ancrage (6).

8. Dispositif de fixation (1) selon la revendication 7, dans lequel le levier (11) et l'enclume d'impact (7) sont agencés sur un côté supérieur de la barre transversale (2) en tant qu'autre côté par rapport à la au moins une cheville d'ancrage (6).

9. Dispositif de fixation (1) selon l'une des revendications 7 ou 8, dans lequel :
- un côté frontal inférieur, tourné vers le tibia et/ou la barre transversale (2), de l'enclume d'impact (7) est ajusté, en particulier chanfreiné, de manière à reposer dans une position introduite du système d'ancrage (4, 5) contre le côté supérieur de la barre transversale (2) pour la fourniture d'une butée (8) afin de délimiter une longueur d'impact de la au moins une cheville d'ancrage (6) ;
- l'enclume d'impact (7) forme à son extrémité inférieure un étage (9) pour une venue en prise de l'extrémité de course (10), de préférence arrondie, du levier (11) afin de repousser le système d'ancrage (4, 5) loin de la barre transversale (2) ;
- le levier (11) est conçu en forme de S et s'étendant essentiellement vers l'avant et est articulé au moyen d'une tige de charnière (12) dans des œillets de charnière correspondants, conçus dans un prolongement et/ou une saillie (13) sur le côté supérieur de la barre transversale (2) ; et
- une extrémité de prise (14) du levier (11) qui se situe à l'opposé de l'extrémité de course (10) est prévue pour l'actionnement de celui-ci.

10. Dispositif de fixation (1) selon l'une des revendications précédentes, dans lequel la barre transversale (2) présente un élément de guidage à son extrémité avant (3) via lequel la barre transversale (2) peut être montée contre la tige principale (H) du système d'alignement de tibia, de manière librement coulissante axialement par rapport à la barre.

11. Dispositif de fixation (1) selon la revendication 10, dans lequel la barre transversale (2) peut être montée contre la tige principale (H) de manière à pouvoir être retirée librement.

12. Dispositif de fixation (1) selon l'une des revendications 10 ou 11, dans lequel, par le montage en coulissement libre axialement par rapport à la barre de la barre transversale (2), une distance entre la au moins une cheville d'ancrage (6) et la tige principale (H) peut être réglée, pour régler une profondeur de coupe.

13. Dispositif de fixation (1) selon l'une des revendications précédentes, dans lequel sont prévues deux chevilles d'ancrage (6) qui se déroulent parallèlement l'une à l'autre, lesquelles sont reliées à l'enclume d'impact (7).
